# EUROPEAN PATENT APPLICATION

(11) **EP 4 342 519 A1**
(43) Date of publication of application: **27.03.2024**
(21) Application number: 22913615.5
(22) Date of filing: 08.10.2022
(51) Int. Cl.: A61N 1/39

(54) **MEDICAL DEVICE**

(30) Priority: 31.12.2021 CN 202111664247
(71) Applicant: Shenzhen Mindray Bio-Medical Electronics Co., Ltd, Shenzhen, Guangdong 518057 (CN)
(72) Inventor: ZOU, Xiaoling, Shenzhen, Guangdong 518057 (CN); ZHANG, Lei, Shenzhen, Guangdong 518057 (CN); JI, Baimiao, Shenzhen, Guangdong 518057 (CN); DING, Yanqiong, Shenzhen, Guangdong 518057 (CN)
(74) Representative: KIPA AB
(86) International application number: PCT/CN2022/123844
(87) International publication number: WO 2023/124351

(57) **Abstract**

A medical device (100, 200, 300), comprising a processor (120, 220, 320) and a display apparatus (110, 210, 310). The processor (120, 220, 320) is connected to the display apparatus (110, 210, 310) to control the display of the display apparatus (110, 210, 310); a display interface of the display apparatus (110, 210, 310) comprises a function display area, the function display area selectively or fixedly displaying a navigation area; the function display area can display a first function page when a display instruction is received; the navigation area simultaneously displays a plurality of labels, the labels comprising at least two of a manual external defibrillator label, an automated external defibrillator label, and a pace-making label; and when one of the plurality of labels is selected, the first function page displays content related to a selected label. The medical device (100, 200, 300) can simultaneously display labels of at least two treatment functions related to defibrillation and/or pace-making, and if any label is selected, a treatment function page corresponding to the label is displayed, so that first-aid personnel can quickly select an appropriate treatment function according to conditions, and can view the corresponding content so as to diagnose and treat a patient in time.

## Description

### TECHNICAL FIELD

The disclosure relates to medical instruments, and more particularly to a medical device.

### BACKGROUND

In pre-hospital emergency situations, the most important life-saving tool for professional emergency personnel is a defibrillation monitor. At present, the vast majority of devices on the market include treatment functions through discharge for short-term fatal heart diseases, such as ventricular fibrillation, atrial fibrillation, or arrhythmia, which functions include such as manual defibrillation, automated external defibrillation, and pacing function. However, in the current device, if a display page of one treatment function should be switched to a display page of another treatment function, the display page related to the one treatment function should be closed and then the display page related to the other treatment function can be reopened, which is very inconvenient to operate.

### SUMMARY

This disclosure is submitted to address at least one of the above problems. According to one aspect of this disclosure, a medical device is provided, including a processor and a display apparatus, wherein the processor is connected with the display apparatus and configured to control the display apparatus for displaying; a display interface of the display apparatus includes a function display area, which is configured to selectively or fixedly display a navigation area, wherein the function display area is further configured to display a first function page, when receiving a display instruction; wherein the navigation area is configured to simultaneously display multiple labels, which include at least two of a manual external defibrillator label, an automated external defibrillator label and a pacing label; wherein the first function page is configured to display content related to a selected label, when selecting one of the multiple labels.

According to another aspect of this disclosure, a medical device is provided, including a processor and a display apparatus, wherein the processor is connected with the display apparatus and configured to control the display apparatus for displaying; a display interface of the display apparatus includes a function display area, which is configured to selectively or fixedly display a navigation area, wherein the function display area is further configured to display a first function page, when receiving a display instruction; wherein the navigation area is configured to simultaneously display multiple labels, which include a first label and a second label; wherein the first function page is configured to display defibrillation energy when selecting the first label, and the first function page is configured to display a pacing rate and/or a pacing current when selecting the second label.

According to another further aspect of this disclosure, a medical device is provided, medical device is provided, including a processor and a display apparatus, wherein the processor is connected with the display apparatus and configured to control the display apparatus for displaying; a display interface of the display apparatus includes a function display area, which is configured to selectively or fixedly display a navigation area, wherein the function display area is further configured to display a first function page, when receiving a display instruction; wherein the navigation area is configured to display a first label; wherein the first function page is configured to display defibrillation energy, as well as a pacing rate and/or a pacing current, when selecting the first label.

According to embodiments of this disclosure, the medical device can simultaneously display labels for at least two treatment functions related to defibrillation and/or pacing. If any label is selected, the corresponding treatment function page can be displayed. Therefore, one treatment function page can be switched to another by simply selecting a label without requiring complex operations of closing the current treatment function page and reopening another treatment function page, which allows emergency personnel to quickly select suitable treatment functions based on a situation and to view corresponding content, so as to timely diagnose and treat patients.

### BRIEF DESCRIPTION OF THE DRAWINGS

The above and other purposes, features, and advantages of this disclosure becomes more apparent, through a more detailed description of the embodiments of this disclosure combining the accompanying drawings. The accompanying drawings are used to provide a further understanding of the embodiments of this disclosure and form a part of the description. The accompanying drawings are used together with the embodiments of this disclosure to explain this disclosure and do not constitute a limitation on this disclosure. In the accompanying drawings, the same reference numerals usually represent the same components or steps.
FIG. 1 is a structural block diagram of a medical device according to an embodiment of this disclosure, as well as an example diagram for a display interface of the medical device.
FIG. 2 is a diagram for an example of a function display area on a display interface of a medical device, according to an embodiment of this disclosure.
FIG. 3 is a diagram for another example of a function display area on a display interface of a medical device, according to an embodiment of this disclosure.
FIG. 4 is a diagram for another further example of a function display area on a display interface of a medical device, according to an embodiment of this disclosure.
FIG. 5 is a diagram for an example of a shortcut operation area shown in FIG. 4.
FIG. 6 is a diagram for an example of a dynamic changing area shown in FIGS. 2 and 4.
FIG. 7 is a diagram for another further example of a function display area on a display interface of a medical device, according to an embodiment of this disclosure.
FIG. 8 is a diagram for an example of a fixed pacing sub-label page or an on-demand pacing sub-label page shown in FIG. 7.
FIG. 9 is diagram for an example of a display interface of the medical device according to an embodiment of this disclosure, wherein the display interface includes a display area for monitoring parameter(s).
FIG. 10 is a diagram for an example of the display area for monitoring parameter(s) shown in FIG.9.
FIG. 11 is diagram for an example of a display interface of the medical device according to an embodiment of this disclosure, wherein the display interface includes a display area for a state bar.
FIG. 12 is a diagram for another further example of a function display area on a display interface of a medical device, according to an embodiment of this disclosure.
FIG. 13 is diagram for an example of a layout of a display area for a state bar, a display area for monitoring parameter(s) and a function display area on a display interface of a medical device, according to an embodiment of this disclosure.
FIG. 14 is diagram for another example of a layout of a display area for a state bar, a display area for monitoring parameter(s) and a function display area on a display interface of a medical device, according to an embodiment of this disclosure.
FIG. 15 is diagram for another further example of a layout of a display area for a state bar, a display area for monitoring parameter(s) and a function display area on a display interface of a medical device, according to an embodiment of this disclosure.
FIG. 16 is diagram for another further example of a layout of a display area for a state bar, a display area for monitoring parameter(s) and a function display area on a display interface of a medical device, according to an embodiment of this disclosure.
FIG. 17 is diagram for an example of a display interface of a medical device according to an embodiment of this disclosure.
FIG. 18 is diagram for another example of a display interface of a medical device according to an embodiment of this disclosure.
FIG. 19 is an enlarged view of a page corresponding to manual defibrillation shown in FIG. 18.
FIG. 20 is diagram for another further example of a display interface of a medical device according to an embodiment of this disclosure.
FIG. 21 is diagram for another further example of a display interface of a medical device according to an embodiment of this disclosure.
FIG. 22 is diagram for another further example of a display interface of a medical device according to an embodiment of this disclosure.
FIG. 23 is diagram for another further example of a display interface of a medical device according to an embodiment of this disclosure.
FIG. 24 is a structural block diagram of a medical device according to another embodiment of this disclosure, as well as an example diagram for a display interface of the medical device.
FIG. 25 is a structural block diagram of a medical device according to another further embodiment of this disclosure, as well as an example diagram for a display interface of the medical device.

### DESCRIPTION OF THE PREFERRED EMBODIMENTS

In order to make the purpose, technical solution, and advantage of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

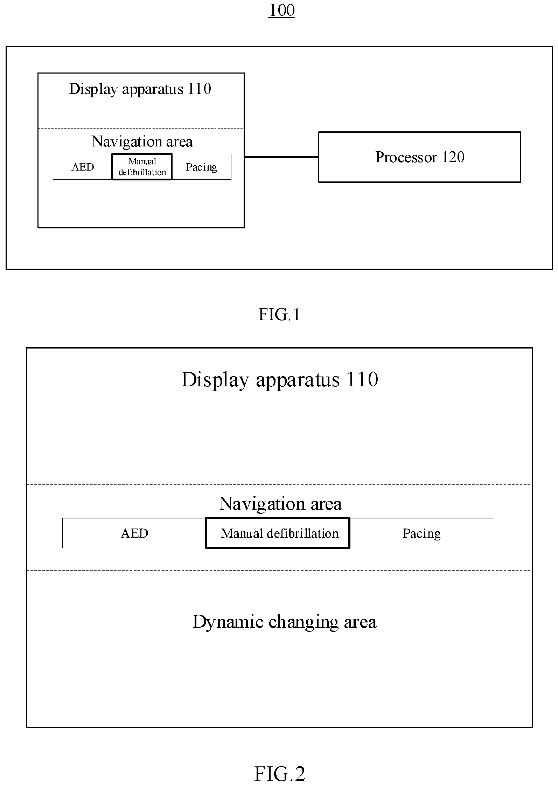

Firstly, a medical device according to an embodiment of this disclosure is described with reference to FIG. 1. FIG. 1 is a structural block diagram of a medical device 100 according to an embodiment of this disclosure, as well as an example diagram for a display interface of the medical device 100. As shown in FIG. 1, the medical device 100 includes a display apparatus 110 and a processor 120. Wherein a display interface of the display apparatus 110 includes a function display area, which selectively or fixedly displays a navigation area. The function display area displays a first function page when receiving a display instruction. The navigation area simultaneously displays multiple labels, which include at least two of a manual external defibrillator label, an automated external defibrillator (AED) label, and a pacing label (as an example shown in FIG. 1, three labels). When selecting one of the multiple labels, the first function page displays content related to the selected label.

In an embodiment of this disclosure, the display interface of the medical device 100 includes a function display area, which is configured to display medical functions of the medical device 100. In one example, the function display area fixedly displays the navigation area. For example, the navigation area can be fixedly displayed at at least one of an upper side, a lower side, a left side, or a right side of the display interface. At this time, regardless of whether the first function page corresponding to the navigation area is opened or closed, the navigation area is permanently displayed on the display interface, achieving an effect of quickly entering the first function page. The navigation area displays multiple labels simultaneously, which relate to a defibrillation treatment function and/or a pacing treatment function. Specifically, these labels include at least two of a manual external defibrillator label, an automated external defibrillator label and a pacing label. When any one of these labels is selected, the function display area receives a display instruction, so as to display the first function page, which displays content related to the selected label.

In another example, the function display area selectively displays the navigation area. In this example, when the function display area receives a display instruction, the first function page is displayed, and the navigation area is displayed when displaying the first function page. The navigation area can be displayed at an upper side, a lower side, a left side, or a right side of the first function page, and can be a part of the first function page, or can be displayed inside other areas of the function display area. In some embodiments, the navigation area can also be opened or closed together with the corresponding first function page. When the first function page is closed, the navigation area is no longer displayed, which never occupies too more display space, allowing the display interface to display more other information. The navigation area simultaneously displays multiple labels, which relate to a defibrillation treatment function and/or a pacing treatment function. Specifically, these labels include at least two of a manual external defibrillator label, an automated external defibrillator label and a pacing label. When any of these labels is selected, the first function page displays content related to the selected label.

In the above two examples, since the navigation area includes at least two labels, when displaying the relevant content of one label in the first function page, if the user wishes to switch to another treatment function, by selecting another label in the navigation area, the first function page can be directly switched to the content related to said another label, without requiring complex operations of closing the current treatment function page and reopening another treatment function page, which allows emergency personnel to quickly select suitable treatment functions based on a situation and to view corresponding content, so as to timely diagnose and treat patients.

In an embodiment of this disclosure, the aforementioned first function page can include the aforementioned navigation area and a dynamic changing area, as shown in FIG. 2. Wherein, when one of the multiple labels is selected, the dynamic changing area displays content related to the selected label. In this embodiment, the content related to the selected label is displayed through the dynamic changing area. The reason that it is called as a dynamic changing area is because the displayed content at a display position of the dynamic changing area changes dynamically due to a change of the selected label. Therefore, the displayed content in this area is dynamically changing.

In an embodiment of this disclosure, the aforementioned labels of the navigation area include three labels, namely the manual external defibrillator label, the automated external defibrillator label and the pacing label. In this embodiment, the navigation area includes the manual external defibrillator label, the automated external defibrillator label and the pacing label. Therefore, when the manual external defibrillator label is selected, the first function page displays content related to the manual defibrillation treatment function. When the automated external defibrillator label is selected, the first function page displays content related to the automated defibrillation treatment function. When the pacing label is selected, the first function page displays content related to the pacing treatment function.

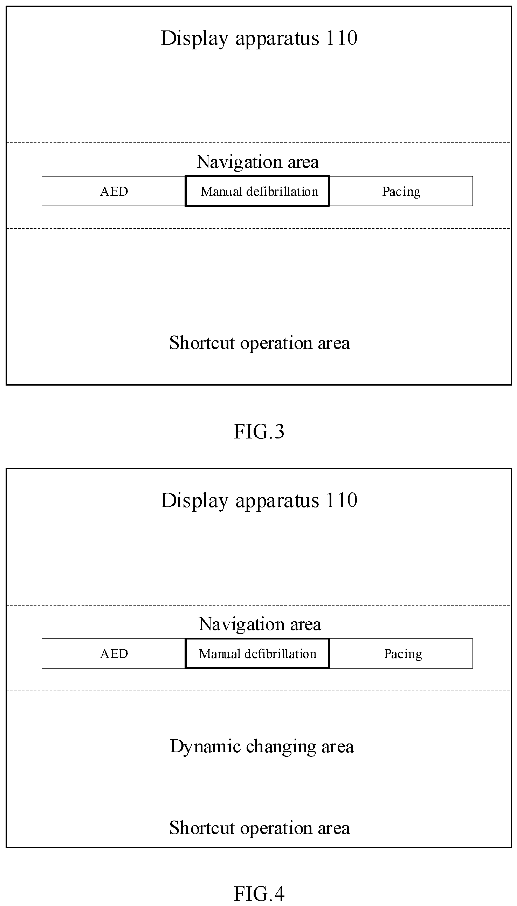

In an embodiment of this disclosure, the first function page further includes a shortcut operation area, as shown in FIG. 3. When one of the multiple labels is selected, the shortcut operation area displays at least an auxiliary treatment key related to the selected label, and the shortcut operation area and the content related to the selected label are displayed on the same screen. In this embodiment, the auxiliary treatment key related to the selected label are displayed through the shortcut operation area, where the auxiliary treatment key is a key that has an auxiliary treatment function for the corresponding treatment function of the selected label, such as a medication record key, an event record key, etc. Based on displaying, on the same screen, the auxiliary treatment key related to the selected label in the shortcut operation area and the content related to the selected label, user (emergency personnel) can view the auxiliary treatment function related to a certain treatment function while viewing the content related to said treatment function, which is more conducive to the diagnosis and treatment of patient.

In an embodiment of this disclosure, the first function page further includes a shortcut operation area, which is displayed on the same screen with the navigation area and the dynamic changing area, as shown in FIG. 4. The shortcut operation area displays at least an auxiliary treatment key related to the selected label. In this embodiment, the aforementioned navigation area, dynamic changing area, and shortcut operation area are displayed on the same screen, allowing user (emergency personnel) to conveniently switch treatment functions through the navigation area, view the currently selected treatment function through the dynamic changing area, and conveniently view auxiliary treatment functions associated with the currently selected treatment function in the shortcut operation area, greatly improving the selection function during patient diagnosis and treatment and the convenience of information viewing. In the example shown in FIG. 4, a layout, in which the navigation area, the dynamic changing area, and the shortcut operation area are arranged side by side along an up-down direction, is used as an example layout. It should be understood that in other examples, the positions of the navigation area, dynamic changing area, and shortcut operation area can also have other layout situations, such as the navigation area, dynamic changing area, and shortcut operation area are arranged along a left-right direction.

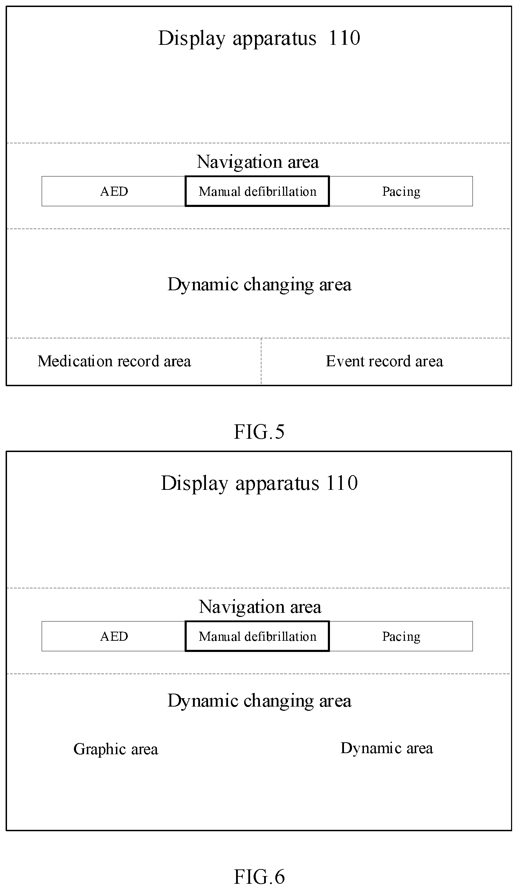

In an embodiment of this disclosure, the aforementioned shortcut operation area can include a medication record area, as shown in FIG. 5. Correspondingly, the auxiliary treatment key displayed inside the shortcut operation area can include a medication record key. The medication record area displays at least one medication record key, and each medication record key displays a medication name and a corresponding dosage of the medication name. In this embodiment, through the medication record key in the medication record area of the shortcut operation area, the user can conveniently view the medication record of the current rescued patient, including the medication name and the corresponding dosage used, which facilitates the user to combine medication and treatment conditions to determine a treatment effect and/or treatment plan.

In an embodiment of this disclosure, different medication record keys can display the same medication name, but the different medication record keys with the same medication name display different medication dosages. In this embodiment, the medication record area of the shortcut operation area includes multiple medication record keys. Different medication record keys may display the same medication name but different dosages. On the one hand, it allows user to more flexibly set multiple treatment plans for using the same medication, and on the other hand, it also allows user to view subtle changes in the medication situation of the rescued person, and more accurately understand the changes in the condition of the rescued patient.

In an embodiment of this disclosure, the aforementioned shortcut operation area can include an event record area, as shown in FIG. 5. Correspondingly, the auxiliary treatment key includes an event record key, the event record area displays at least one event record key, and each event record key displays a rescue event. Wherein, the rescue event can include at least one of oxygen inhalation, ventilation, intubation, neck support, hemostasis, bandaging, and fixation. In this embodiment, through the event record key in the event record area of the shortcut operation area, the user can conveniently view the rescue event of the rescued patient, making it easy for the user to determine the treatment effect of the rescued patient and/or the next treatment plan based on the rescue event.

In an embodiment of this disclosure, when a manual external defibrillator label or an automated external defibrillator label is selected, the first function page, which displays content related to the selected label, includes a graphic area and a dynamic area, as shown in FIG. 6. In this embodiment, when the user selects a label corresponding to the defibrillation treatment function, the content related to the defibrillation treatment function is displayed through the graphic area and the dynamic area. Wherein, the graphic area display at least one of an operation graphic, a pressing graphic, a warning graphic and a rescue timing graphic. The dynamic area can dynamically display treatment indication information (such as low impedance) and/or treatment suggestion information (such as "please connect electrode correctly") and/or provide treatment operation entry (such as charging, discharging, etc.). The dynamic area can include a defibrillation statistic area, which displays treatment statistic information (such as shock count). Based on information displayed inside the graphic area and the dynamic area, the user can view many aspects of the defibrillation treatment process in real-time, which provides great convenience for the defibrillation treatment of the rescued patient.

In an embodiment of this disclosure, the first function page can display or close the defibrillation statistic area and/or the shortcut operation area based on a user operation. In this embodiment, a display of the defibrillation statistic area and/or a display of the shortcut operation area is user selectable, and the user can select to display or close the defibrillation statistic area and/or the shortcut operation area, so as to satisfy requirements of different scenarios.

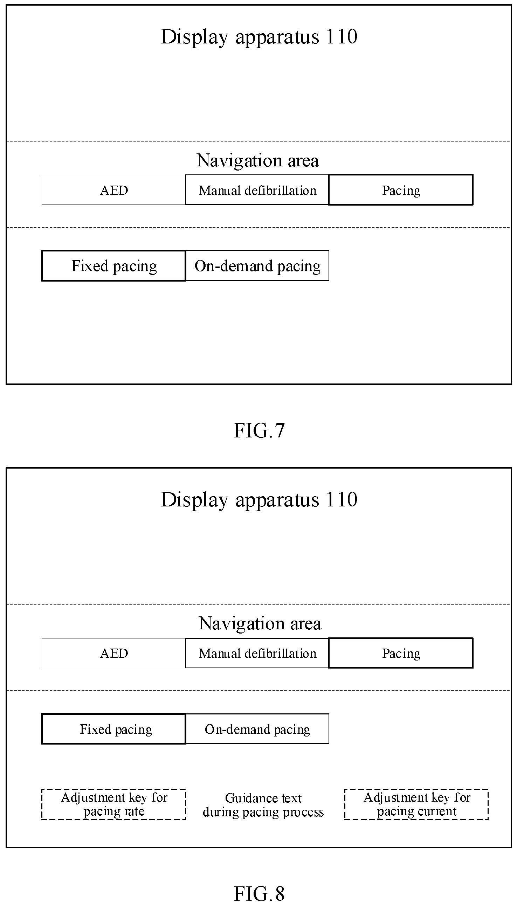

In an embodiment of this disclosure, when the pacing label is selected, the first function page includes multiple sub-label pages, which include a fixed pacing sub-label page and an on-demand pacing sub-label page which are switchable between each other, as shown in FIG. 7. In this embodiment, the pacing treatment function further provides two sub-functions, namely fixed pacing and on-demand pacing. These two sub-functions are displayed through two sub-label pages that can be switched between each other. The user can select one of the sub-functions according to his/her requirement to display the corresponding sub-label page for that sub-function. Of course, when displaying a sub-label page, user can also easily switch from it to another sub-label page.

In an embodiment of this disclosure, each of the multiple sub-label pages includes an adjustment key for a pacing rate, an adjustment key for a pacing current, and guidance text during a pacing process, as shown in FIG. 8. Based on this, user can conveniently adjust the pacing rate and the pacing current as needed, and can also understand the pacing treatment situation through the guidance text during the pacing process (such as "pacing in progress").

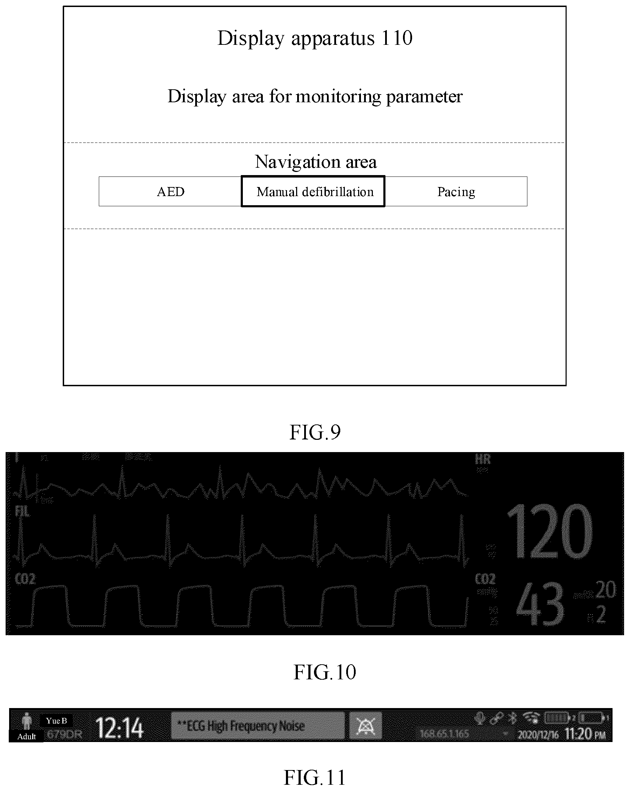

In an embodiment of this disclosure, the display interface may also include a display area for monitoring parameter(s), which is configured to display waveform(s) and/or value(s) of the monitoring parameter(s), as shown in FIG. 9. In this embodiment, in addition to displaying the aforementioned treatment functions related to defibrillation and/or pacing, the display interface of the medical device 100 also displays monitoring parameter data, facilitating user to view both monitoring and treatment information at the same time, which is more conducive for rescue personnel to evaluating treatment quality and determining the next step of treatment plan based on the evaluation results.

In an embodiment of this disclosure, the aforementioned display area for monitoring parameter(s) can include a graphic display area and a numerical display area, as shown in FIG. 10. The graphic display area is configured display waveform(s) of the monitoring parameter(s) (such as electrocardiogram waveform(s), filtered electrocardiogram waveform(s), and carbon dioxide CO₂ waveform(s), shown in FIG. 10), and the numerical display area is configured to display value(s) of the monitoring parameter(s) (such as heart rate value(s) and carbon dioxide value(s) shown in FIG. 10). In this embodiment, by displaying the waveform(s) of monitoring parameter(s) in the graphic display area, the user can view a trend over time of physiological parameter(s) of the rescued patient. By displaying the value(s) of monitoring parameter(s) in the numerical display area, the user can view real-time value(s) of the physiological parameter(s) of the rescued patient. Therefore, combined with the display of the graphic display area and the display of the numerical display area, user can view the detailed physiological parameter(s) of the rescued patient during the treatment process in real time, which helps to monitor the rescued patient in real time and evaluate the treatment process.

In an embodiment of this disclosure, the aforementioned display area for monitoring parameter(s) can display waveforms of at least three monitoring parameters, which allows user to view a large number of monitoring parameter situations and facilitate comprehensive evaluation of the condition of the rescued patient. Alternatively, the display area for monitoring parameter(s) can display at least electrocardiogram waveforms, blood oxygen waveforms, and carbon dioxide waveforms, allowing user to view changes in electrocardiogram signals, blood oxygen signals, and carbon dioxide signals of the rescued patient. The changes in these three parameters are usually important for the emergency process, so displaying the waveforms of these three parameters simultaneously is beneficial for emergency personnel to monitor and evaluate the rescued patient during the emergency process.

In an embodiment of this disclosure, the display area for monitoring parameter(s) can switch from currently displayed waveform(s) to another target waveform(s) based on a user operation. In this embodiment, the display area for monitoring parameter(s) is user interactive, so that user can select to display desired waveform(s) of monitoring parameter(s) according to his/her requirements. In one example, the user can click on waveforms of one monitoring parameter currently displayed to switch to waveforms of another monitoring parameter. For example, clicking on a CO₂ waveform area can switch from CO₂ waveforms to another waveforms (such as blood oxygen waveforms, etc., which can be set by the user). Clicking on the waveform area for waveform switching is more intuitive and can save screen display area. It can be understood that the waveform switching control can also be set separately in other positions on the screen instead of being set in the waveform area.

In the embodiment of this disclosure, the display interface may also include a display area for a state bar, which displays at least one of patient information, department, license plate, rescue time, alarm information, and device state. Based on the content displayed inside the display area for a state bar, user can view information related to patient, device, alarm, etc. The following is an exemplary description in conjunction with FIG. 11.

As shown in FIG. 11, the display area for a state bar includes a patient information area, a display area for a department number and a license plate number, rescue time, an alarm information area, and a device state area. The patient information area needs to display at least a graphic or text for a patient type (such as "adult" shown in FIG. 11). The display area for the department number and the license plate number should display at least one piece of information (such as license plate number information shown in FIG. 11). The alarm information area includes a physiological alarm, a technical alarm, and indication information (such as physiological alarm information for ECG shown in FIG. 11). Wherein, the physiological alarm includes several types of information, such as a high-level physiological alarm, an intermediate physiological alarm, and a low-level physiological alarm, as well as several alarm states, such as alarm sound pause, alarm sound turnoff, alarm pause, alarm turnoff, and alarm reset. At least two of the physiological alarm, the technical alarm, and the indication information are displayed inside the same area through a polling manner. Optionally, the three types of information are displayed inside a unified area through a polling manner, achieving a streamlined display of alarm information and providing more display space for information display in other areas. In an example, when the three types of information are displayed inside a unified area through a polling manner, a priority order for the display can be the physiological alarm, the technical alarm, and then the indication information. Wherein, a priority order for the display of the physiological alarm can be the high-level physiological alarm, the intermediate physiological alarm, and then the low-level physiological alarm.

The device state area can include a battery state graphic, a state graphic for a network connection, a record state graphic, a camera state graphic, an entry to a connection state and operation, an entry to system time and setting, and so on. Wherein, the battery state graphic can include an AC graphic, a graphic which indicates the battery is normal, a charging graphic, a battery level graphic, a graphic which indicates the battery is not in place, a graphic which indicates the battery is abnormal, and so on. The state graphic for the network connection can include Bluetooth, Wi-Fi, wired network, and so on. The device state area must generally display the battery state graphic and the system time. It can be understood that the above information can be displayed through graphics, colors, texts, and other means.

In an embodiment of this disclosure, the display area for a state bar is a global state display area, and the interface in any rescue mode can display information for at least one area of the display area for a state bar. In different rescue modes, the content of these areas can be combined arbitrarily. For example, only the patient information area and device state area can be displayed. For further example, only the alarm information area and the patient information area can be displayed, or optionally other combination of areas can be displayed. In addition, the layout and display for the information inside the display area for a state bar can be arbitrary. For example, the patient type is on the left, the battery state and system time is on the right, and the alarm information is in the middle, all of which can be adjusted on demand.

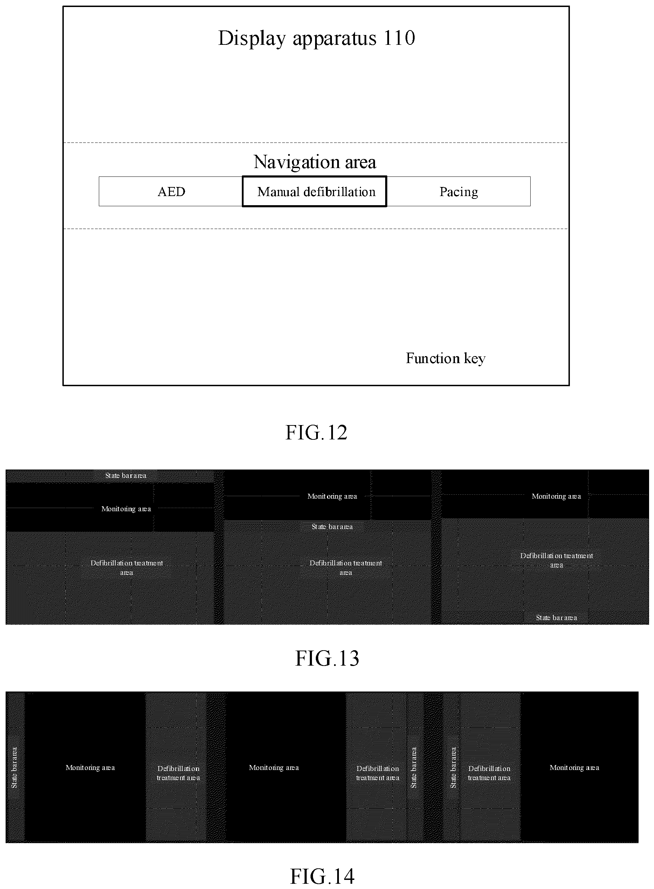

In the embodiment of this disclosure, the function display area may also include a function key area, as shown in FIG. 12. Wherein, the aforementioned function key area can be configured to display a function key of the medical device 100, such as a main menu key. When the main menu key is selected, at least one clinical operation key, which is non-high frequency, is displayed. Due to the infrequent use of some non-high frequency clinical operations, their corresponding keys can be hidden in the main menu key for saving the display space and providing more space for the display of other important information, so that important information (such as the content displayed inside the display area for monitoring parameter(s) and defibrillation related function pages mentioned earlier) can be displayed more clearly. The main menu key can serve as an entry to a global operation, and can be displayed on any interface during the rescue process, and can be kept at a bottom right position of the screen for user to click and operate.

In addition to the main menu key, shortcut function keys, such as a record key and a scoring key, as well as auxiliary function keys, such as an ultrasound key, a twelve-lead electrocardiogram analysis key, a traumatic brain injury monitoring key, and a video key, can also be included. In the several example interface diagrams described below, these auxiliary function keys are not displayed because they are covered by other contents in the function display area (such as the content corresponding to defibrillation/pacing labels). Of course, in other examples, when displaying the function page inside the function display area, these keys can also be left, such as being displayed below the medication record area and the event record area. In some embodiments, the function keys are also used for user to conveniently open the desired function page. After selecting a specific function key, the display interface displays the corresponding function page, such as the first function page. In some embodiments, only one treatment function key can be set, and once the treatment function key is selected, the first function page can be accessed. User can select to set a default display of content corresponding to the manual external defibrillator label, automated external defibrillator label, pacing label, or other treatment related labels. In other embodiments, the function key area includes the navigation area, where the labels in the navigation area themselves are also function keys. When the user selects a label/function key, the first function page is opened and the corresponding content of the label/function key is displayed.

In an embodiment of this disclosure, the function display area covers waveforms and/or numerical values of some monitoring parameters displayed inside the display area for monitoring parameter(s) when displaying the function page. Therefore, the function display area and the display area for monitoring parameter(s) can be integrated with each other and then displayed. In addition, the function pages can all include a close key to close the currently displayed function page. After the function page is closed, the display interface can re-display the covered waveforms and/or numerical values of some monitoring parameters. In some embodiments, when the function display area displays the function page, the display area for monitoring parameter(s) can also adjust formats of the waveforms and/or values of the monitoring parameters, such as sizes, layouts, etc., so that when the function page is opened, as much monitoring information as possible can be displayed inside the remaining limited display space. Of course, in other embodiments, when the function display area displays the function page, the display area for monitoring parameter(s) can also directly turn off the display of the monitoring parameters in the corresponding area of the function page, in order to save display resources.

In an embodiment of this disclosure, the display layout of the display area for a state bar, the display area for monitoring parameter(s), and the function display area can be default or user-defined. The display area for a state bar can be located at an upper side, a lower side, a left side or a right side of the display area for monitoring parameter(s) and the function display area. The following is an exemplary description in conjunction with FIG.S 13 to 16.

As shown in FIG. 13, in this example, the display area for a state bar, the display area for monitoring parameter(s) (referred to as monitoring area), and the function display area (referred to as defibrillation treatment area) can be arranged in an up-middle-down layout. For example, the display area for a state bar is located above the monitoring area, and the monitoring area is located above the defibrillation treatment area. Alternatively, the monitoring area is located above the display area for a state bar, and the display area for a state bar is located above the defibrillation treatment area. For further example, the monitoring area is located above the defibrillation treatment area, and the defibrillation treatment area is located above the display area for a state bar.

As shown in FIG. 14, in this example, the display area for a state bar, the display area for monitoring parameter(s) (referred to as monitoring area), and the function display area (referred to as defibrillation treatment area) can be arranged in a left-middle-right layout. For example, the display area for a state bar is on the left of the monitoring area, and the monitoring area is on the left of the defibrillation treatment area. Alternatively, the monitoring area is on the left of the defibrillation treatment area, and the defibrillation treatment area is on the left of the display area for a state bar. For further example, the display area for a state bar is on the left of the defibrillation treatment area, and the defibrillation treatment area is on the left of the monitoring area.

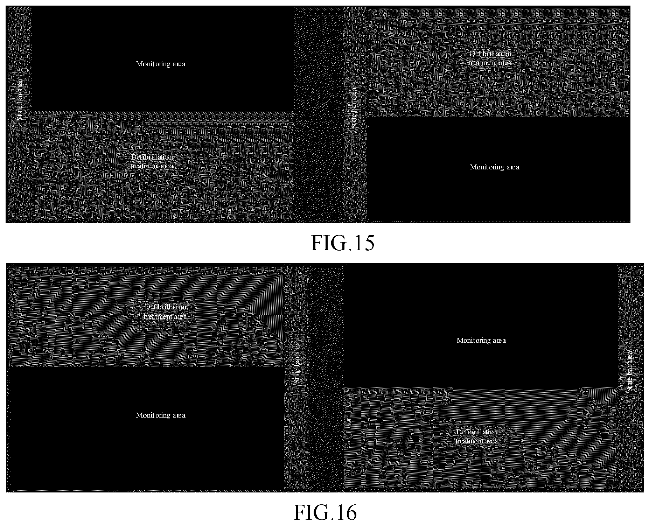

As shown in FIG. 15, in this example, the display area for a state bar, the display area for monitoring parameter(s) (referred to as monitoring area), and the function display area (referred to as defibrillation treatment area) can include a left-right and up-down layout. For example, the display area for a state bar is on the left of the monitoring area and the defibrillation treatment area, while the monitoring area is above the defibrillation treatment area. Alternatively, the display area for a state bar is located on the left of the monitoring area and the defibrillation treatment area, while the defibrillation treatment area is located above the monitoring area.

As shown in FIG. 16, in this example, the display area for a state bar, the display area for monitoring parameter(s) (referred to as monitoring area), and the function display area (referred to as defibrillation treatment area) can include a left-right and up-down layout. For example, the display area for a state bar is located on the right of the monitoring area and the defibrillation treatment area, while the monitoring area is located below the defibrillation treatment area. Alternatively, the display area for a state bar is located on the right of the monitoring area and the defibrillation treatment area, while the defibrillation treatment area is located below the monitoring area.

In an embodiment of this disclosure, relatively less important display areas can be invoked for display through a user operation. For example, the defibrillation statistic area, the shortcut operation area (such as medication record area), and waveforms of at least one monitoring parameter mentioned above can be invoked and displayed through a user operation (such as gesture, for example, single-finger sliding, double-finger sliding, etc.; as well as clicking a key to invoke). For example, they can be invoked in a pop-up style. This can provide greater display space for important display areas to better display important information.

In an embodiment of this disclosure, the content of a single large area can be reorganized with the content of another large area. For example, at least one of the navigation area, the dynamic changing area, and the shortcut operation area mentioned earlier can be displayed inside the display area for monitoring parameter(s), and/or at least one of the navigation area, the dynamic changing area, and the shortcut operation area can be displayed inside another area among the navigation area, the dynamic changing area, and the shortcut operation area. That is to say, in this embodiment, different display areas on the display interface can be fused for display, which increases a display area of the function page and enable it to display more treatment information.

In the embodiment of this disclosure, the display apparatus 110 may also include physical keys for safe backup in areas outside the display interface, such as a defibrillation key, an ultrasound key, an electrocardiogram key, a discharge key, an energy addition/subtraction key, an AED key, etc. Physical keys can reduce the risk of accidental touch by user, thereby reducing the risk of incorrect treatment.

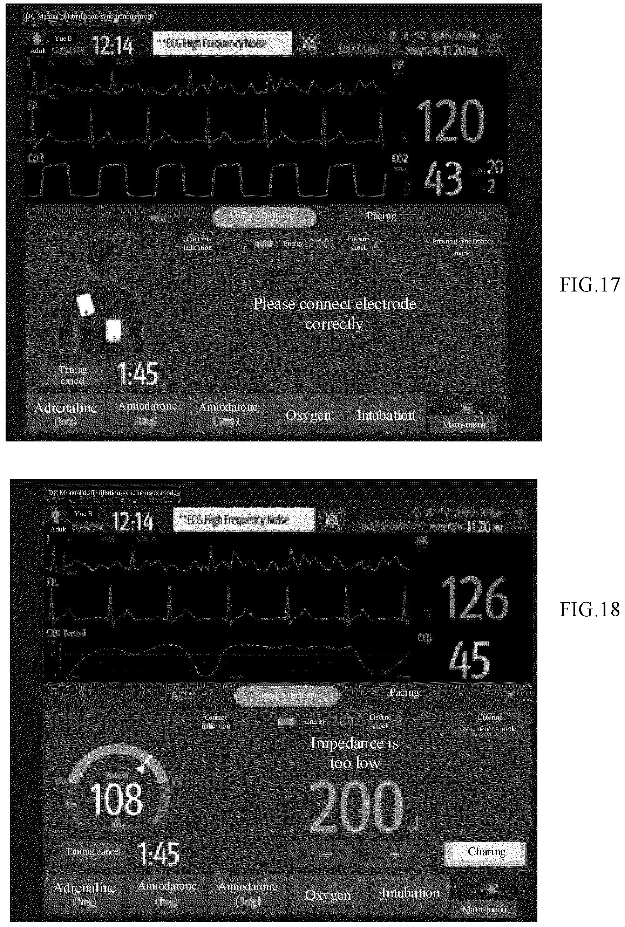

Below, a more detailed example diagram including a display interface of a medical device 100 according to an embodiment of this disclosure, which can be a combination of several embodiments mentioned above, is illustrated in conjunction with FIGS. 17 to 23.

FIG. 17 is diagram for an example of a page displayed when a manual external defibrillator label in a navigation area of the medical device 100 is selected, according to an embodiment of this disclosure. As shown in FIG. 17, the display interface consists of a display area for a state bar, a display area for monitoring parameter(s), and a function display area, from top to bottom. Wherein, the display area for a state bar displays patient information, time, alarm information, device power, and other contents. The display area for monitoring parameter(s) displays waveforms and values of several monitoring parameters. The function display area currently displays content related to manual defibrillation, including a graphic area, a dynamic area, a medication record area, and an event record area. Wherein, the graphic area displays an operation graphic (with electrode patches attached to a human body graphic) and a rescue timing graphic (1 minute and 45 seconds). The dynamic area displays treatment suggestion information ("Please connect electrode correctly"). The dynamic area also displays a defibrillation statistic area, which displays treatment statistic information (shock count 2, defibrillation energy 200J). The medication record area displays three medication record keys, namely, adrenaline (1mg), amiodarone (Img), and amiodarone (3mg). Due to that the guidelines defines the dosages of adrenaline and lidocaine used in the rescue process, the medication record area at least displays incidental dosages of adrenaline and lidocaine. In additional, interval countdown indication for the adrenaline is also displayed. In addition, although not shown in the drawings, other available medication records can include medications, such as amiodarone, atropine, adenosine, dopamine, and aminophylline, along with relevant information such as dosages. The event record area displays two event of oxygen and intubation. In addition, the function display area also displays a function key, that is, the main menu key.

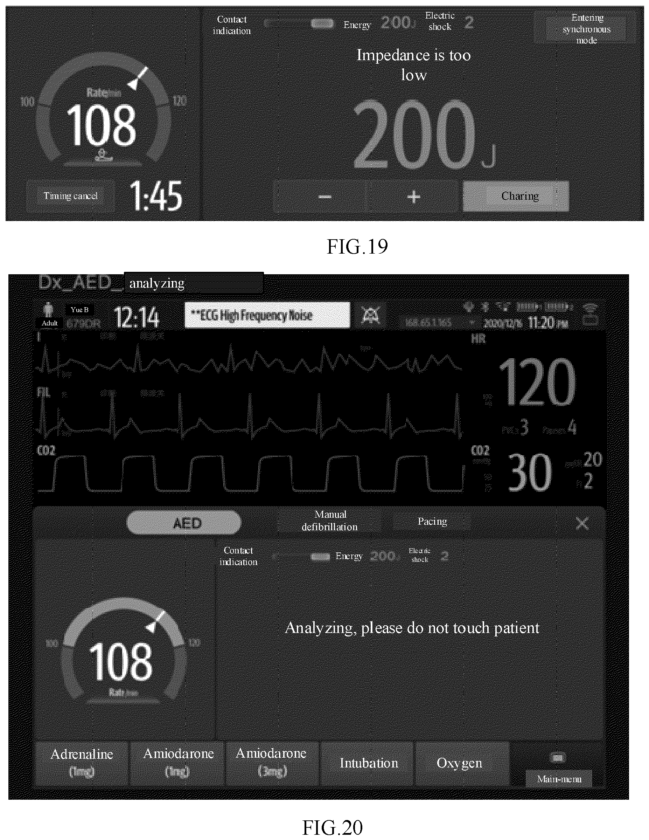

FIG. 18 is diagram for another example of a page displayed when a manual external defibrillator label in a navigation area of the medical device 100 is selected, according to an embodiment of this disclosure. As shown in FIG. 18, the display interface consists of a display area for a state bar, a display area for monitoring parameter(s), and a function display area, from top to bottom. Wherein, the display area for a state bar displays patient information, time, alarm information, device power, and other contents. The display area for monitoring parameter(s) displays waveforms and values of several monitoring parameters. The function display area currently displays content related to manual defibrillation, including a graphic area, a dynamic area, a medication record area, and an event record area. Wherein, the graphic area displays a pressing graphic (pressing frequency 108) and a rescue timing graphic (1 minute and 45 seconds). The dynamic area displays treatment indication information ("impedance is too low") and treatment operation entry ("charging"). The dynamic area also displays a defibrillation statistic area, which displays treatment statistic information (shock count 2, defibrillation energy 200J). The medication record area displays three medication record keys, namely adrenaline (1mg), amiodarone (1mg), and amiodarone (3mg). The event record area displays two events of oxygen and intubation. In addition, the function display area also displays a function key, that is, the main menu key. FIG. 19 is an enlarged view of the function display area in FIG. 18, which can be combined to provide a clearer view of the content displayed inside the function display area.

FIG. 20 is diagram for another example of a page displayed when an AED label in a navigation area of the medical device 100 is selected, according to an embodiment of this disclosure. As shown in FIG. 20, the display interface consists of a display area for a state bar, a display area for monitoring parameter(s), and a function display area, from top to bottom. Wherein, the display area for a state bar displays patient information, time, alarm information, device power, and other contents. The display area for monitoring parameter(s) displays waveforms and values of several monitoring parameters. The function display area currently displays content related to automated defibrillation, including a graphic area, a dynamic area, a medication record area, and an event record area. Wherein, the graphic area displays a pressing graphic (pressing frequency 108). The dynamic area displays treatment indication information ("Analyzing, please do not touch patient"). The dynamic area also displays a defibrillation statistic area, which displays treatment statistic information (shock count 2, defibrillation energy 200J). The medication record area displays three medication record keys, namely adrenaline (1mg), amiodarone (1mg), and amiodarone (3mg). The event record area displays two events of oxygen and intubation. In addition, the function display area also displays a function keys, that is, the main menu key.

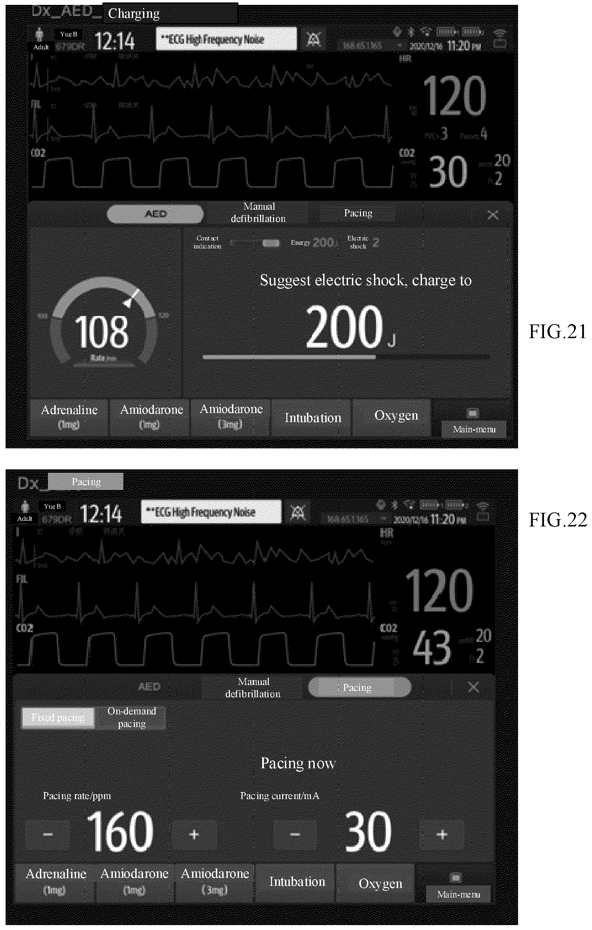

FIG. 21 is diagram for another example of a page displayed when an AED label in a navigation area of the medical device 100 is selected, according to an embodiment of this disclosure. As shown in FIG. 21, the display interface consists of a display area for a state bar, a display area for monitoring parameter(s), and a function display area, from top to bottom. Wherein, the display area for a state bar displays patient information, time, alarm information, device power, and other contents. The display area for monitoring parameter(s) displays waveforms and values of several monitoring parameters. The function display area currently displays content related to automated defibrillation, including a graphic area, a dynamic area, a medication record area, and an event record area. Wherein, the graphic area displays a pressing graphic (pressing frequency 108). The dynamic area displays treatment suggestion information ("Suggest electric shock, charge to 200J"). The dynamic area also displays a defibrillation statistic area, which displays treatment statistic information (shock count 2, defibrillation energy 200J). The medication record area displays three medication record keys, namely adrenaline (1mg), amiodarone (1mg), and amiodarone (3mg). The event record area displays two events of oxygen and intubation. In addition, the function display area also displays a function keys, that is, the main menu key.

FIG. 22 is diagram for an example of a page displayed when a pacing label in a navigation area of the medical device 100 is selected, according to an embodiment of this disclosure. As shown in FIG. 22, the display interface consists of a display area for a state bar, a display area for monitoring parameter(s), and a function display area, from top to bottom. Wherein, the display area for a state bar displays patient information, time, alarm information, device power, and other contents. The display area for monitoring parameter(s) displays waveforms and values of several monitoring parameters. The function display area currently displays content related to pacing. The function page corresponding to the pacing label further includes two sub-label pages, namely a fixed pacing sub-label page and an on-demand pacing sub-label page. The fixed pacing sub-label page is currently displayed. The sub-label page includes an adjustment key for a pacing rate (such as plus and minus keys on the left and right sides of 160 shown in FIG. 22), an adjustment key for a pacing current (such as plus and minus keys on the left and right sides of 30 shown in FIG. 22), and guidance text during a pacing process (such as "Pacing in Progress" shown in FIG. 22). In addition, the sub-label page also includes a medication record area and an event record area. The medication record area displays three medication record keys, namely adrenaline (1mg), amiodarone (1mg), and amiodarone (3mg). The event record area displays two events of oxygen and intubation. In addition, the function display area also displays a function keys, that is, the main menu key.

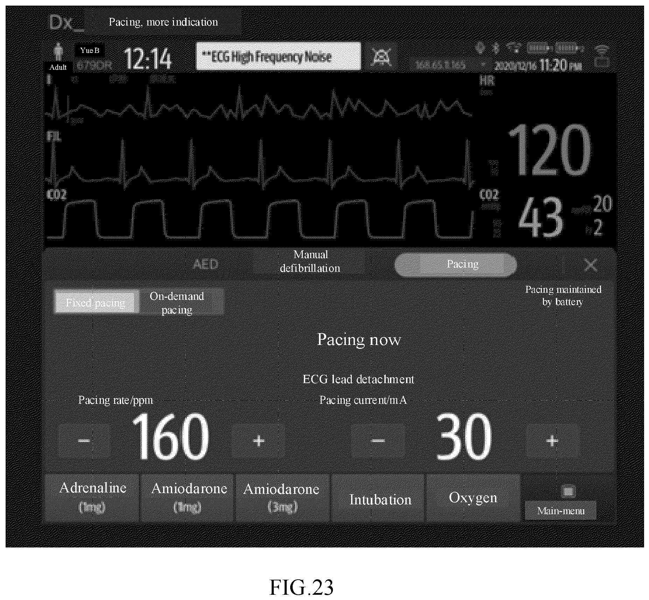

FIG. 23 is diagram for another example of a page displayed when a pacing label in a navigation area of the medical device 100 is selected, according to an embodiment of this disclosure. As shown in FIG. 23, the display interface consists of a display area for a state bar, a display area for monitoring parameter(s), and a function display area, from top to bottom. Wherein, the display area for a state bar displays patient information, time, alarm information, device power, and other contents. The display area for monitoring parameter(s) displays waveforms and values of several monitoring parameters. The function display area currently displays content related to pacing. The function page corresponding to the pacing label further includes two sub-label pages, namely a fixed pacing sub-label page and an on-demand pacing sub-label page. The fixed pacing sub-label page is currently displayed. The sub-label page includes an adjustment key for a pacing rate (such as plus and minus keys on the left and right sides of 160 shown in FIG. 23), an adjustment key for a pacing current (such as plus and minus keys on the left and right sides of 30 shown in FIG. 23), and guidance text during a pacing process (such as "Pacing is in progress" shown in FIG. 23), as well as alarm information of "ECG lead detachment". In addition, the sub-label page also includes a medication record area and an event record area. The medication record area displays three medication record keys, namely adrenaline (1mg), amiodarone (1mg), and amiodarone (3mg). The event record area displays two events of oxygen and intubation. In addition, the function display area also displays a function keys, that is, the main menu key.

The above example illustrates the medical device 100 according to the embodiment of this disclosure. Based on the above description, according to the embodiment of this disclosure, the medical device 100 can simultaneously display labels for at least two treatment functions related to defibrillation and/or pacing. If any label is selected, the corresponding treatment function page can be displayed. Therefore, one treatment function page can be switched to another by simply selecting a label without requiring complex operations of closing the current treatment function page and reopening another treatment function page, which allows emergency personnel to quickly select suitable treatment functions based on a situation and to view corresponding content, so as to timely diagnose and treat patients.

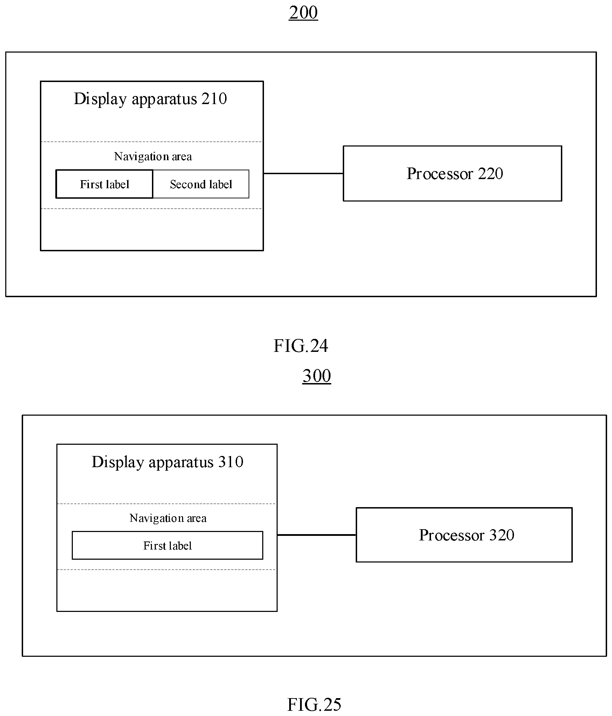

The following describes a medical device 200 according to another embodiment of this disclosure, in conjunction with FIG. 24. As shown in FIG. 24, the medical device 200 includes a display apparatus 210 and a processor 220, wherein the processor 220 is connected with the display apparatus 210, and configured to control the display apparatus for displaying 210. A display interface of the display apparatus 210 includes a function display area, which selectively or fixedly displays a navigation area. The function display area is further configured to display a first function page, when receiving a display instruction. The navigation area simultaneously displays multiple labels, which include a first label and a second label. The first function page is configured to display defibrillation energy when selecting the first label, and the first function page is configured to display a pacing rate and/or a pacing current when selecting the second label.

The medical device 200 according to the embodiment of this disclosure is generally similar to the medical device 100 according to the embodiment of this disclosure mentioned earlier, with following difference. On the display interface of the display apparatus 110 of the medical device 100 according to the embodiment of this disclosure, the navigation area of the function display area includes at least two labels of an automated external defibrillator label, a manual external defibrillator label, and a pacing label, wherein the automated external defibrillator label and the manual external defibrillator label are separate and independent labels. However, on the display interface of the display apparatus 210 of the medical device 200 according to this embodiment of this disclosure, when the first label is selected among the multiple labels in the navigation area of the function display area, defibrillation energy is displayed, and when the second label is selected among the multiple labels in the navigation area of the function display area, a pacing rate and/or a pacing current are/is displayed. That is to say, on the display interface of the display apparatus 210 of the medical device 200, the navigation area of the function display area does not distinguish the automated external defibrillator label and the manual external defibrillator label, but instead displays the defibrillation energy by selecting the first label. It can also be understood that on the display interface of the display apparatus 210 of the medical device 200, the function display area combines the corresponding contents of the automated defibrillation and the manual defibrillation into one page for display. As the structure and display interface of the medical device 100 are described in detail in the previous description, for the sake of brevity, no further details are given here, only some main display contents are described. Those skilled in the art can combine the previous description to understand the structure and display interface of the medical device 200 according to the embodiment of this disclosure.

In an embodiment of this disclosure, the first function page includes a navigation area and a dynamic changing area. When the first label is selected, the dynamic changing area displays defibrillation energy. When the second label is selected, the dynamic changing area displays a pacing rate and/or a pacing current.

In an embodiment of this disclosure, the first label is a defibrillation label, and the second label is a pacing label, wherein the defibrillation label corresponds to a manual external defibrillation function and/or an automated external defibrillation function.

In an embodiment of this disclosure, the display interface of the display apparatus includes a display area for monitoring parameter(s) and a function display area, wherein the display area for monitoring parameter(s) is configured to display waveform(s) and/or value(s) of the monitoring parameter(s). The function display area is configured to display a second function page that is different from the first function page when receiving a display instruction. The function display area is further configured to cover waveforms and/or numerical values of several monitoring parameters, which are displayed inside the display area for monitoring parameter(s), when displaying the first function page and/or the second function page. The first function page and the second function page respectively includes a close key, which is configured to close a currently displayed first or second function page. After the first and second function pages are closed, the display interface re-displays the covered waveforms and/or values of said monitored parameters.

Based on the above description, according to the embodiment of this disclosure, the medical device 200 can simultaneously display labels for at least two treatment functions related to defibrillation and pacing. If any label is selected, the corresponding treatment function page can be displayed. Therefore, one treatment function page can be switched to another by simply selecting a label without requiring complex operations of closing the current treatment function page and reopening another treatment function page, which allows emergency personnel to quickly select suitable treatment functions based on a situation and to view corresponding content, so as to timely diagnose and treat patients.

The following describes a medical device 300 according to another embodiment of this disclosure, in conjunction with FIG. 25. As shown in FIG. 25, medical device 300 includes a display apparatus 310 and a processor 320, wherein the processor 320 is connected with the display apparatus 310, and configured to control the display apparatus for displaying 310. A display interface of the display apparatus 310 includes a function display area, which selectively or fixedly displays a navigation area. The function display area is further configured to display a first function page, when receiving a display instruction. The navigation area is configured to at least display a first label. When the first label is selected, the first function page displays defibrillation energy, as well as a pacing rate and/or a pacing current.

The medical device 300 according to the embodiment of this disclosure is generally similar to the medical device 100 according to the embodiment of this disclosure mentioned earlier, with following difference. On the display interface of the display apparatus 110 of the medical device 100 according to the embodiment of this disclosure, the navigation area of the function display area includes at least two labels of an automated external defibrillator label, a manual external defibrillator label, and a pacing label, wherein the automated external defibrillator label, the manual external defibrillator label and the pacing label are separate and independent labels. However, on the display interface of the display apparatus 310 of the medical device 300 according to the embodiment of this disclosure, the navigation area of the function display area includes at least one label, which displays the defibrillation energy, pacing rate, and/or pacing current when selected. That is to say, on the display interface of the display apparatus 310 of the medical device 300, the navigation area of the function display area does not distinguish the automated external defibrillator label, the manual external defibrillator label, and the pacing label. Instead, by selecting one label, the defibrillation function related content and pacing function related content are displayed simultaneously. It can also be understood that on the display interface of the display apparatus 310 of the medical device 300, the function display area combines the corresponding contents of the automated defibrillation, the manual defibrillation, and the pacing into one page for display. As the structure and display interface of the medical device 100 are described in detail in the previous description, for the sake of brevity, no further details are given here, only some main display contents are described. Those skilled in the art can combine the previous description to understand the structure and display interface of the medical device 300 according to the embodiment of this disclosure.

Based on the above description, according to the embodiment of this disclosure, the medical device 300 can display labels for defibrillation and pacing related treatment functions when selected, without requiring complex operations of closing the current treatment function page and reopening another treatment function page, which allows emergency personnel to quickly select suitable treatment functions based on a situation and to view corresponding content, so as to timely diagnose and treat patients.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific disclosure and design constraints of the technical solution. Professional technicians may use different methods to implement the described functions for each specific disclosure, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some features can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various features of this disclosure are sometimes grouped together into a single embodiment, diagram, or description thereof. However, the method of this disclosure should not be interpreted as reflecting the intention that the claimed protection of this disclosure requires more features than those explicitly recorded in each claim. More precisely, as reflected in the corresponding claims, the inventive point is that the corresponding technical problem can be solved with less than all features of a single disclosed embodiment. Therefore, the claims following the specific implementation method are explicitly incorporated into the specific implementation method, where each claim itself serves as a separate embodiment of this disclosure.

Technicians in this field can understand that, in addition to mutual exclusion between features, any combination can be configured to combine all features disclosed in this disclosure (including accompanying claims, abstract, and drawings), as well as all processes or units of any method or device so disclosed. Unless otherwise explicitly stated, each feature disclosed in this disclosure (including accompanying claims, abstract, and accompanying drawings) may be replaced by alternative features that provide the same, equivalent, or similar purpose.

In addition, those skilled in the art can understand that although some embodiments described herein include certain features rather than other features included in other embodiments, the combination of features of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Technicians in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate the present application rather than make a limitation of this disclosure, and those skilled in the art can design alternative embodiments without departing from the scope of the attached claims. In the claims, any reference symbol between parentheses should not be constructed as a restriction on the claims. The word "comprising" does not exclude the presence of components or steps not listed in the claims. The words "a" or "an" before a component do not exclude the existence of multiple such components. This application can be implemented with the help of hardware consisting of several different components and with the help of appropriately programmed computers. Among the claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A medical device, **characterized in that**, comprising a processor and a display apparatus; wherein:
the processor is connected with the display apparatus and configured to control the display apparatus for displaying;
a display interface of the display apparatus comprises a function display area, which is configured to selectively or fixedly display a navigation area;
the function display area is further configured to display a first function page, when receiving a display instruction;
the navigation area is configured to simultaneously display multiple labels, which comprise at least two of a manual external defibrillator label, an automated external defibrillator label and a pacing label; and
the first function page is configured to display content which is related to a selected label, when selecting one of the multiple labels.

2. The medical device according to claim 1, **characterized in that**, the first function page comprises the navigation area and a dynamic changing area;
wherein the dynamic changing area is configured to display the content which is related to the selected label, when selecting one of the multiple labels.

3. The medical device according to claim 1, **characterized in that**, the multiple labels comprise three labels, which are the manual external defibrillator label, the automated external defibrillator label and the pacing label.

4. The medical device according to claim 1, **characterized in that**, the first function page comprises a shortcut operation area;
wherein the shortcut operation area is configured to at least display an auxiliary treatment key which is related to the selected label, when selecting one of the multiple labels;
wherein the shortcut operation area and the content which is related to the selected label are displayed on a same screen.

5. The medical device according to claim 2, **characterized in that**, the first function page further comprises a shortcut operation area, which is displayed on a same screen with the navigation area and the dynamic changing area;
wherein the shortcut operation area is configured to at least display an auxiliary treatment key which is related to the selected label.

6. The medical device according to claim 4 or 5, **characterized in that**, the shortcut operation area comprises a medication record area, the auxiliary treatment key comprises medication record key(s), wherein the medication record area is configured to display at least one medication record key, and each medication record key is configured to display one medication name and a dosage which corresponds to the medication name.

7. The medical device according to claim 6, **characterized in that**, different medication record keys are capable of displaying a same medication name, and different medication record keys with the same medication name display different dosages.

8. The medical device according to claim 5, **characterized in that**, the shortcut operation area comprises an event record area, the auxiliary treatment key comprises event record key(s), the event record area is configured to display at least one event record key, and each event record key is configured to display one rescue event.

9. The medical device according to claim 8, **characterized in that**, the rescue event comprises at least one of oxygen inhalation, ventilation, intubation, neck support, hemostasis, bandaging and fixation.

10. The medical device according to claim 1, **characterized in that**, the first function page, which is configured to display the content which is related to the selected label, comprises a graphic area and a dynamic area, when selecting the manual external defibrillator label or the automated external defibrillator label.

11. The medical device according to claim 10, **characterized in that**, the graphic area is configured to display at least one of an operation graphic, a pressing graphic, a warning graphic and a rescue timing graphic.

12. The medical device according to claim 10, **characterized in that**, the dynamic area is configured to dynamically display treatment indication information and/or treatment suggestion information, and/or to provide an entry to a treatment operation.

13. The medical device according to claim 10, **characterized in that**, the dynamic area comprises a defibrillation statistic area, which is configured to display treatment statistic information.

14. The medical device according to claim 13, **characterized in that**, the first function page is capable of displaying or closing the defibrillation statistic area and/or a shortcut operation area according to a user operation.

15. The medical device according to claim 1, **characterized in that**, the first function page comprises multiple sub-label pages, when selecting the pacing label; wherein the multiple sub-label pages comprise a fixed pacing sub-label page and an on-demand pacing sub-label page, which pages are capable of being switched between each other.

16. The medical device according to claim 15, **characterized in that**, each sub-label page comprises an adjustment key for a pacing rate, an adjustment key for a pacing current, and guidance text during a pacing process.

17. The medical device according to claim 1 or 2, **characterized in that**, the display interface further comprises a display area for monitoring parameter(s), which is configured to display waveform(s) and/or value(s) of the monitoring parameter(s).

18. The medical device according to claim 17, **characterized in that**, the display area for monitoring parameter(s) comprises a graphic display area and a numerical display area; wherein the graphic display area is configured to display the waveform(s) of the monitoring parameter(s), and the numerical display area is configured to display the value(s) of the monitoring parameter(s).

19. The medical device according to claim 17, **characterized in that**, the display area for monitoring parameter(s) is further configured to at least display waveforms of three monitoring parameters; or
the display area for monitoring parameter(s) is further configured to at least display electrocardiogram waveform(s), blood oxygen waveform(s) and carbon dioxide waveform(s).

20. The medical device according to claim 18, **characterized in that**, the display area for monitoring parameter(s) is capable of switching from currently displayed waveform(s) to other target waveform(s) according to a user operation.

21. The medical device according to claim 17, **characterized in that**, the display interface further comprises a display area for a state bar, which display area is configured to display at least one of patient information, department, license plate, rescue time, alarm information and device state.

22. The medical device according to claim 21, **characterized in that**, the display area for a state bar is located at an upper side, a lower side, a left side or a right side of the display area for monitoring parameter(s) and the function display area.

23. The medical device according to any one of claims 17-22, **characterized in that**, the first function page further comprises a shortcut operation area;
wherein at least one of the navigation area, the dynamic changing area and the shortcut operation area is capable of being displayed inside the display area for monitoring parameter(s); and/or
at least one of the navigation area, the dynamic changing area and the shortcut operation area is capable of being displayed inside another area among the navigation area, the dynamic changing area and the shortcut operation area.

24. The medical device according to claim 5, **characterized in that**, the navigation area, the dynamic changing area and the shortcut operation area are arranged side by side in a left-right direction or in an up-down direction.

25. The medical device according to any one of claims 1-24, **characterized in that**, the function display area further comprises a function key area, which is configured to display a function key of the medical device; wherein the display interface is configured to display a function page, when selecting a predetermined function key; wherein the function page comprises the first function page.

26. The medical device according to claim 25, **characterized in that**, the function display area is configured to, when displaying the function page, cover waveforms and/or values of some monitoring parameters, which waveforms and/or values are displayed inside a display area for monitoring parameter(s).

27. The medical device according to claim 26, **characterized in that**, the function page further comprises a close key, which is configured to close a currently displayed function page;
wherein the display interface is further configured to re-display the covered waveforms and/or values of some monitoring parameters, after closing said function page.

28. A medical device, **characterized in that**, comprising a processor and a display apparatus; wherein:
the processor is connected with the display apparatus and configured to control the display apparatus for displaying;
a display interface of the display apparatus comprises a function display area, which is configured to selectively or fixedly display a navigation area;
the function display area is further configured to display a first function page, when receiving a display instruction;
the navigation area is configured to simultaneously display multiple labels, which comprise a first label and a second label;
wherein the first function page is configured to display defibrillation energy when selecting the first label, and the first function page is further configured to display a pacing rate and/or a pacing current when selecting the second label.

29. The medical device according to claim 28, **characterized in that**, the first function page comprises the navigation area and a dynamic changing area;
wherein the dynamic changing area is configured to display the defibrillation energy, when selecting the first label; and the dynamic changing area is further configured to display the pacing rate and/or the pacing current, when selecting the second label.

30. The medical device according to claim 28, **characterized in that**, the first label is a defibrillation label, and the second label is a pacing label; wherein the defibrillation label corresponds to a manual external defibrillation function and/or an automated external defibrillation function.

31. The medical device according to claim 27, **characterized in that**, the display interface of the display apparatus further comprises a display area for monitoring parameter(s);
the display area for monitoring parameter(s) is configured to display waveform(s) and/or value(s) of the monitoring parameter(s);
the function display area is capable of displaying a second function page which is different from the first function page, when a display instruction is received; wherein the function display area is further configured to, when displaying the first function page and/or the second function page, cover waveforms and/or values of some monitoring parameters, which are displayed inside the display area for monitoring parameter(s); and
the first function page and the second function page respectively comprises a close key which is configured to close the first function page or the second function page that is currently displayed; the display interface is further configured to re-display the covered waveforms and/or values of some monitoring parameters, after closing the first function page and the second function page.

32. A medical device, **characterized in that**, comprising a processor and a display apparatus;
wherein the processor is connected with the display apparatus and configured to control the display apparatus for displaying;
a display interface of the display apparatus comprises a function display area, which is configured to selectively or fixedly display a navigation area;
the function display area is further configured to display a first function page, when receiving a display instruction;
the navigation area is configured to at least display a first label;
the first function page is configured to display defibrillation energy, as well as a pacing rate and/or a pacing current, when selecting the first label.
